# EUROPEAN PATENT APPLICATION

(11) **EP 2 762 877 A1**
(43) Date of publication of application: **06.08.2014**
(21) Application number: 13405013.7
(22) Date of filing: 31.01.2013
(51) Int. Cl.: G01N 33/00

(54) **Calibration of a chemical sensor in a portable electronic device**

(71) Applicant: Sensirion AG, 8712 Stäfa (CH)
(72) Inventor: Mayer, Felix, 8712 Stäfa (CH)
(74) Representative: Detken, Andreas

(57) **Abstract**

In a method for calibrating a portable first electronic device (1) comprising a first chemical sensor, a determination is carried out whether the first electronic device is located near a second electronic device comprising a second chemical sensor. If this is the case and if optionally other criteria are fulfilled, readings of the first and second chemical sensor are compared. Subject to the comparison, calibration values for the first chemical sensor are derived.

## Description

### TECHNICAL FIELD

The present invention relates to methods of determining calibration data for chemical sensors in electronic devices, to a corresponding system and to corresponding software.

### PRIOR ART

Portable electronic devices such as mobile phones, tablet computers, notebook computers etc. have become ubiquitous in everyday life. Such devices are nowadays equipped with a multitude of sensors, including gyroscopes, acceleration sensors, magnetic field sensors, proximity sensors, cameras, GPS modules etc.

It would be desirable to integrate further sensors into portable electronic devices, in particular, sensors that are sensitive to chemical analytes. Such sensors will in the following be called "chemical sensors". In particular, semiconductor sensors are known for this purpose. Such sensors have a sensitive layer with at least one electrical property that changes in the presence of one or more analytes. In some embodiments, the sensitive layer must be heated to a desired operational temperature. For instance, metal-oxide sensors are known; these sensors are to be operated at elevated temperatures of a few hundred degrees Celsius. In order to achieve these temperatures in the sensitive layer, a heater thermally coupled to the sensitive layer may be heated prior to and/or during taking a sensor reading. However, semiconductor sensors and in particular metal-oxide sensors may suffer from drift even when the sensor is not operated and even in the absence of any chemical stimulus to the sensor. This may also be true for other types of sensors. Drift may be understood as a variation in the sensor signal over time under identical environmental conditions in the absence of any chemical stimulus. Drift may impact the transfer function of the sensor in various forms, including an offset drift representing an additive component to the sensor signal and a sensitivity drift affecting a gradient of the transfer function. Any drift in turn may impact the accuracy of the sensor reading. The sensor should therefore be recalibrated from time to time to account for the drift.

### SUMMARY OF THE INVENTION

In a first aspect, the present invention provides a method for operating a portable electronic device comprising a chemical sensor in which an impact of a drift of the chemical sensor on a reading of the chemical sensor may be reduced, the method having the features of claim 1. Further embodiments of the invention are laid down in the dependent claims.

Accordingly, a method for calibrating a portable first electronic device comprising a first chemical sensor is provided. The method comprises:
determining whether the first electronic device is located near a second electronic device comprising a second chemical sensor;
comparing at least one reading of the first chemical sensor and at least one reading of the second chemical sensor obtained while the first electronic device is near the second electronic device; and
subject to the comparison, deriving at least one calibration value for the first chemical sensor.

The calibration value that is obtained as a result of the method according of the invention may then be employed to modify a subsequent reading of the first chemical sensor. This modification may be done locally on the portable first electronic device, or the modification may for each sensor reading be carried out by a remote determination unit communicably connected to the portable first electronic device via a network.

In the method, the readings of two sensors that can be assumed to be in the same chemical environment are compared, and the result of this comparison is taken into account for recalibrating the first chemical sensor. For determining whether or not the two sensors actually are in the same chemical environment, various criteria may be employed. The first and most important criterion is that the devices that contain the sensors are sufficiently close to one another. Additional criteria may be employed, as will be discussed further below.

If the two sensors sense the same chemical environment, they should yield the same readings if properly calibrated. If the readings differ significantly, this may be an indication that at least one of the sensors is miscalibrated and should be recalibrated. Recalibration is carried out by deriving at least one new calibration value for this sensor. Many possibilities exist for how to derive the new calibration value(s). For instance, if it is known that the second sensor is properly calibrated (e.g., if the second sensor is a reference sensor having a known precision), the calibration value(s) of the first sensor may be adjusted such that the reading of the first sensor will be substantially identical to the reading of the second sensor. Further examples will be discussed below.

The first and second chemical sensors may be of the same or different types. Each of the sensors may be sensitive to one or more chemical analytes. There should be at least one common analyte to which both the first and the second sensor are sensitive. Even though water may in principle be considered to be a chemical analyte, in the context of the present invention, water, and in particular water vapor, is preferably not considered to be a chemical analyte. In other words, preferably a humidity sensor is not to be considered a chemical sensor. The first and/or second chemical sensor preferably is arranged inside a housing of the respective electronic device. An opening may be provided in the housing for exposing the chemical sensor to a fluid to be analyzed.

The method is preferably computer-implemented and fully automated. In particular, at least the steps of comparing readings and deriving calibration values are preferably carried out automatically and without user intervention.

The readings of the first and second sensor to be compared may be obtained in at least one of the following manners: According to a first possibility, readings that have been obtained in the course of other measurements in a certain time span before and/or after it was actually determined that the two devices are close to one another may be included in the comparison. In addition or in the alternative, the first and/or second electronic device may be actively triggered to carry out a measurement with its chemical sensor, in response to the determination that the first electronic device is located near the second electronic device. This may be done fully automatically, or it may be done by informing the user that a second device is nearby, and instructing the user to manually trigger a measurement by one or both of the devices. The measurement and/or the comparison may be made subject to further conditions, e.g., to the condition that other parameters confirm that the two devices are likely to sense the same chemical environment. This will be explained in more detail below.

In the simplest case, the first and second electronic devices may be considered to be "near" or "in proximity" to one another when a user of the devices indicates so. For instance, the determination whether the first electronic device is located near the second electronic device may comprise receiving user input to at least one of the first electronic device and the second electronic device, the user input indicating that the first electronic device is located near the second electronic device. The user input may, for instance, include one or more of the following: pressing a key or a key combination; typing a command; audio input by speaking a command into a microphone of one of the electronic devices; etc. The user input may contain an indication of the distance between the devices. For instance, an application program (app) or operating system service executed in the electronic device may request the user to confirm that the two devices are in the same room, or that they are at a distance of less than a certain number of meters of each other, or that they are placed side by side etc.

According to another possibility, proximity of the electronic devices is determined automatically. In this case, the first and second electronic devices may be considered to be "near" or "in proximity" to one another when they are within a certain predefined, fixed or variable spatial range as determined by an automatic determination method. The exact spatial extent of this range may vary and depends on the method by which proximity of the devices is detected. Various such methods may be employed.

For instance, each of the first electronic device and the second electronic device may comprise a short-range communication module, and the determination whether the first electronic device is located near the second electronic device may comprise detecting whether the short-range communication module of the first electronic device and the short-range communication module of the second electronic device are within an operating range of each other. In other words, at least one of the first and second electronic devices monitors whether it is within the operating range of the short-range communication module of another electronic device. When this is the case, it can be concluded that the electronic devices must be located close to one another. The short-range communication module may be, for instance, a Bluetooth module, an infrared module, or a near-field communication (NFC) module such as an RFID module. The short-range communication module may also be a WLAN module, in particular, according to standard IEEE 802.11, configured for peer-to-peer communication. A short-range communication module typically has a limited operational range of less than approximately 100 m, the exact range depending on whether the module is operated indoors or outdoors and on the topography of the environment. In some embodiments (like with typical NFC modules) the range may be less than 1 m or even less than, say, 10 cm. A short-range communication module preferably enables direct communication between the devices (peer-to-peer communication), without employing a separate, dedicated network access point such as a WLAN router. Some short-range communication modules enable the determination of signal strength of other short-range communication modules within their operational range. In this case, at least one of the first and second electronic devices may be configured to measure the signal strength of the short-range communication module of the other electronic device and may employ the measured signal strength to estimate the distance between the devices and to determine whether the devices are located close to one another.

According to another possibility, each of the first electronic device and the second electronic device comprises a wireless network communication module, and the determination whether the first electronic device is located near the second electronic device comprises detecting whether the first electronic device and the second electronic device are located within the same cell of a wireless network. For instance, the wireless network may be a WLAN/Wi-Fi™ network. If the WLAN network is operated in ad hoc mode, the first electronic device and the second electronic device may be considered to be located within the same cell of the network if they are able to communicate peer-to-peer, as described above. In this regard, the above definitions of proximity are partially redundant. If, on the other hand, the WLAN network is operated in infrastructure mode, i.e., if the WLAN network has a least one dedicated access point such as a router or a repeater, which serves as a bridge to another network infrastructure, in particular, a wired network infrastructure, the first and second electronic device may be considered to be located within the same cell of the wireless network if they communicate via the same access point (e.g., via the same router or the same repeater). In other embodiments, the wireless network may be a wireless telephony network, e.g., allowing data communication based on a UMTS or a GPRS standard. Two electronic devices may then be considered to be located within the same cell of the wireless network if they communicate through the same base station. While such cells can be rather large, it may be sufficient for recalibration purposes to know that both electronic devices are located within the same cell if the electronic devices are operated outdoors.

In another possibility, the first electronic device comprises a network communication module for communication with a network, and the determination whether the first electronic device is located near the second electronic device comprises:
transmitting data from the first electronic device to a remote server, the data containing network and/or position information relating to the first electronic device;
on the remote server, determining a position of the first electronic device based on the transmitted data.
   The transmitted data may contain direct position information, such as information determined by a geolocation sensor, e.g., a GPS sensor. In this case, the position of the first electronic device can be derived directly from the position data. In the alternative or in addition, the transmitted data may contain network information. Such information may include identifiers of one or more particular WLAN networks that are "seen" by the electronic device, i.e. of one or more WLAN networks in whose operational range the electronic device is located. Such identifiers may include: IP address and/or MAC address of a network device, in particular, of a network access point, and the SSID of the network. In addition, ancillary information such as WLAN signal strength may be transmitted. These data may be compared with data collected in a database in which such data is correlated with geolocation information. Services operating in this manner are known as "Wi-Fi positioning systems". Also hybrid positioning systems are known and may be employed, combining the above-mentioned technologies such as GPS and Wi-Fi positioning with further technologies. In this manner, the position of the first electronic device can be determined with high precision, indoors or outdoors, and compared to the position of the second electronic device.

The position of the second electronic device may be determined in a similar manner, or it may be known from other sources. For instance, the second electronic device may be a stationary device whose location is known and stored in a database. In particular, the second electronic device may be a reference station, and the second sensor may accordingly be a reference sensor having a known precision. For instance, the second electronic device may be a monitoring station operated by a government agency or a private contractor for monitoring air pollution. Such stations will often contain much more accurate chemical sensors than portable electronic devices such as smartphones or tablet computers.

The various above-described methods for determining whether the first electronic device is located near the second electronic device may also be combined. For instance, if one of the above-described methods indicates that the two devices are near one another, this hypothesis may be confirmed by one or more of the other methods. In particular, if one of the automatic methods of determining proximity indicates that the first and second electronic device are close to one another, this may trigger a message to a user of the first and/or second electronic device that a nearby device was found, and one or both the devices may then request user confirmation that the devices are indeed close to each other.

As mentioned above, the above-described method rests on the assumption that the chemical environment of the first and second sensor is substantially the same. A key criterion that is employed in determining whether the environment is the same is proximity of the devices containing the sensors. However, this criterion may not always be sufficient. For instance, it is readily conceivable that the first device is kept in a user's pocket, whereas the second device is lying open on a table in the same room. In such situations, the chemical environments of the devices may be significantly different despite the fact that the devices are close to one another. The method may therefore employ additional criteria that are indicators of the environment of at least one of the devices. In particular, the method may comprise:
obtaining context information about the first electronic device and/or the second electronic device;
from the context information, determining whether the first and the second sensor are in substantially the same chemical environment; and
comparing at least one reading of the first chemical sensor and at least one reading of the second chemical sensor obtained while the first electronic device is not only near the second electronic device, but also while the first chemical sensor is substantially in the same chemical environment as the second chemical sensor.

In other words, a recalibration is carried out only if the context information indicates that the first and second sensor are in the same chemical environment.

The context information may, for instance, include at least one of the following:
humidity data;
temperature data;
pressure data;
linear and/or rotational acceleration data;
magnetometer data;
brightness data;
image data;
acoustic data;
data from at least one further chemical sensor of the first electronic device;
position information; and
network information about a network to which the first and/or second electronic device is communicably attached.

In particular, humidity data may be obtained from humidity sensors of the first and second electronic device. Each humidity sensor is preferably disposed in a location that ensures that it senses the same environment as the associated chemical sensor. In particular, the humidity sensor is preferably disposed behind the same opening of the housing of the first electronic device, in particular, side by side with the chemical sensor. Many chemical sensors, in particular, semiconductor sensors are cross-sensitive to humidity, and humidity data are preferably used to correct a reading of the first chemical sensor. However, humidity data may also be used to determine whether the first electronic device and the second electronic device sense the same environment, since in this case the humidity readings obtained by both devices should be substantially the same. If the humidity readings are significantly different, this may be used as an indication that the chemical environments of the first and second device are different. As a consequence, the entire recalibration procedure may be stopped, or the user may be advised to ensure that the first and the second electronic device have the same environment.

Likewise, temperature data obtained from temperature sensors of the first and second electronic device may be used to determine whether the two devices are in a location with substantially the same temperature. If this is not the case, this may indicate that the environments of the first and second sensors are different, with the same consequences as above.

In analogy, acceleration data obtained from acceleration sensors of the first and second electronic device may indicate that the first and second electronic device are not handled in the same manner, e.g., that one electronic device is being carried around or wildly shaken while the other electronic device is placed at rest. If the data indicate that the handling conditions of the first and second electronic device are significantly different, the recalibration procedure may be stopped, or the user may be advised to ensure identical handling conditions of the two devices.

Furthermore, acceleration and/or magnetometer data may be used to determine a spatial orientation of the first and second electronic device. Based on orientation information, the user may, e.g., be advised to put the two electronic devices in the same orientation to ensure identical measuring conditions, or otherwise the recalibration procedure may be stopped.

Brightness may be used to determine whether the lighting conditions are substantially the same for the first and second electronic device, and/or acoustic data may be used to determine whether the acoustic environment is essentially the same. Significantly different lighting conditions or acoustic signals may indicate that the devices are not in the same environment, with the same consequences as above.

Image data may be used to determine whether the first and second electronic device record images of the same environment. Incompatible images may indicate that the devices are not in the same environment or are handled differently, with the same consequences as above.

The devices may comprise additional chemical sensors, which may be sensitive to the same or different analytes than the first and second chemical sensors, or the first and/or second sensors may have a plurality of cells. Signals of such additional sensors or of selected cells may be employed to obtain direct indications of the chemical environment of the first and second device.

The context information may further include position information for the first and/or second electronic device and/or network information about a network to which the first electronic device is communicably attached. Such information allows a cross-check with other methods whether the first and second electronic devices are close to one another.

There may be prior knowledge about the accuracy of the two sensors, and this prior knowledge may be employed. For instance, it may be known that the second sensor is a reference sensor whose reading is accurate to within a certain narrow range, or it may be known that one of the sensors has not been operated for an extended period of time or has been exposed to a poisonous or otherwise incompatible environment, which would make it highly likely that recalibration is necessary and that the reading of the sensor without recalibration would be inaccurate. "Poisonous" gases are gases that lead to short- or long-term sensor damage, which reduces sensor accuracy. Also, sensors that are not frequently used are known to lose accuracy compared to regularly used sensors. In order to take such factors into account, each of the first chemical sensor and the second chemical sensor may be assigned an accuracy indicator. This indicator may relate to a known or estimated accuracy of the respective sensor. It may be a single value, e.g., a number between 0 and 1, or a more complex data structure, e.g., an accuracy vector, the vector elements relating to different parameters that are relevant to accuracy such as design accuracy, sensor age, and/or sensor history (e.g., frequency of usage, last usage, usage conditions during the last measurements, last reconditioning, last recalibration etc.). The at least one calibration value for the first chemical sensor may then be determined subject to the accuracy indicators associated with the first and second electronic sensor. For instance, if the accuracy indicators indicate that the first sensor is likely to have a higher accuracy than the second sensor, no recalibration of the first sensor might be carried out at all. If the accuracy indicators indicate that both the first sensor and the second sensor have a similar expected accuracy, recalibration of both sensors may be carried out such that the sensor readings after recalibration will be the average of the readings before recalibration, etc.

The calibration values (which may also be called compensation values) that are derived by the present method may include any parameter related to the transfer function of the sensor. The transfer function relates the concentration of an analyte to which the first chemical sensor is sensitive to a sensor reading. In particular, the calibration values may include any of the following:
an offset parameter (offset compensation value) related to an offset reading in the absence of an analyte to which the first chemical sensor is sensitive; and
a sensitivity parameter related to a sensitivity of the first sensor to a concentration of at least one analyte to which the first chemical sensor is sensitive.

Of course, more complex combinations of calibration values are possible, including cross-sensitivity parameters between different analytes.

The portable first electronic device may be one of the following, without limitation: a mobile phone, a handheld computer, an electronic reader, a tablet computer, a game controller, a pointing device, a photo or a video camera, and a computer peripheral. The second electronic device may also be a portable electronic device, including any of the device types mentioned above, or may be a stationary electronic device.

In another aspect, the present invention provides a method of determining calibration data for electronic devices, each electronic device being equipped with at least one chemical sensor, which method may be carried out by a server that is located remotely from the electronic devices and communicably connected to the electronic devices via a network. The method comprises:
receiving data through a network, the data containing network and/or position information relating to at least one electronic device;
determining a location of each device for which data is received, based on the received data;
determining at least one indicator whether any two or more electronic devices are in substantially the same chemical environment, in particular, located near one another;
receiving, through the network, readings of the chemical sensors of the electronic devices for which the indicator indicates that they are in substantially the same chemical environment;
subject to the readings, deriving calibration data for at least one of the electronic devices.

The method may further comprise sending the derived calibration data to at least one of the electronic devices or to a remote determination unit communicably connected to at least one of the electronic devices via a network.

The same considerations apply to this method as for the method for operating a portable electronic device that is discussed above. In particular, the same methods may be employed for determining whether two or more electronic devices are located near one another as discussed above. The method may optionally comprise triggering at least one of the electronic devices that are near one another to carry out a measurement with their chemical sensors, so as to obtain a reading. As discussed above, context information may be obtained about at least one of the electronic devices, and the further calibrations steps may be carried out subject to the context information. As discussed above, at least one of the electronic devices may be a reference station equipped with a reference sensor.

In a related aspect, the present invention provides a system for determining calibration data for electronic devices equipped with at least one chemical sensor, the system comprising:
a network communication module for communicably attaching the system to a network;
a locating module for receiving data through a network, the data containing network and/or position information relating to at least one electronic device, and for determining a location of each device for which data is received based on the received data;
a matching module for determining at least one indicator whether any two or more electronic devices are substantially in the same chemical environment, in particular, near one another; and
a calibration module for receiving readings of the chemical sensors of the electronic devices for which the indicator indicates that they are substantially in the same chemical environment and, subject to the readings, deriving calibration data for at least one of the electronic devices.

The system may comprise a database for storing the locations of the electronic device. The system may further comprise a sending module for sending the derived calibration data to at least one of the electronic devices or to a remote determination unit communicably connected to at least one of the electronic devices via a network.

The same considerations apply for the system as for the methods discussed above. The modules of the system may be implemented partially or fully in software that is executed on a general-purpose processor of the system. The system may be embodied by a server or a server cluster.

In yet another aspect, the present invention provides a computer program code element that carries out central parts of the methods described above when executed in a processor of a system for determining calibration data. The computer program element comprises computer-implemented instructions to cause a processor to carry out a particular method. It can be provided in any suitable form, including source code or object code. In particular, it can be stored on a computer-readable medium or embodied in a data stream. The data stream may be accessible through a network such as the Internet.

Accordingly, the present invention further relates to a program element comprising computer code that, when executed in a processor, carries out the following method:
receiving data through a network, the data containing network and/or position information relating to at least one electronic device;
determining a location of each device for which data is received, based on the received data;
determining at least one indicator whether any two or more electronic devices are in substantially the same chemical environment, in particular, located near one another;
receiving, through the network, readings of the chemical sensors of the electronic devices for which the indicator indicates that they are in substantially the same chemical environment;
subject to the readings, deriving calibration data for at least one of the electronic devices.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a mobile phone equipped with a chemical sensor;
- Fig. 2: shows a highly schematic block diagram of the mobile phone of Figure 1;
- Fig. 3: shows a highly schematic top view of a sensor chip of a chemical sensor;
- Fig. 4: shows a highly schematic cut through an individual sensor cell of the sensor chip of Fig. 3;
- Fig. 5: shows an illustration of how several electronic devices may be connected to a server via a network;
- Fig. 6: shows a highly schematic block diagram of a server; and
- Fig. 7: is a schematic flow diagram illustrating an exemplary embodiment of a method for determining calibration values.

### DESCRIPTION OF PREFERRED EMBODIMENTS

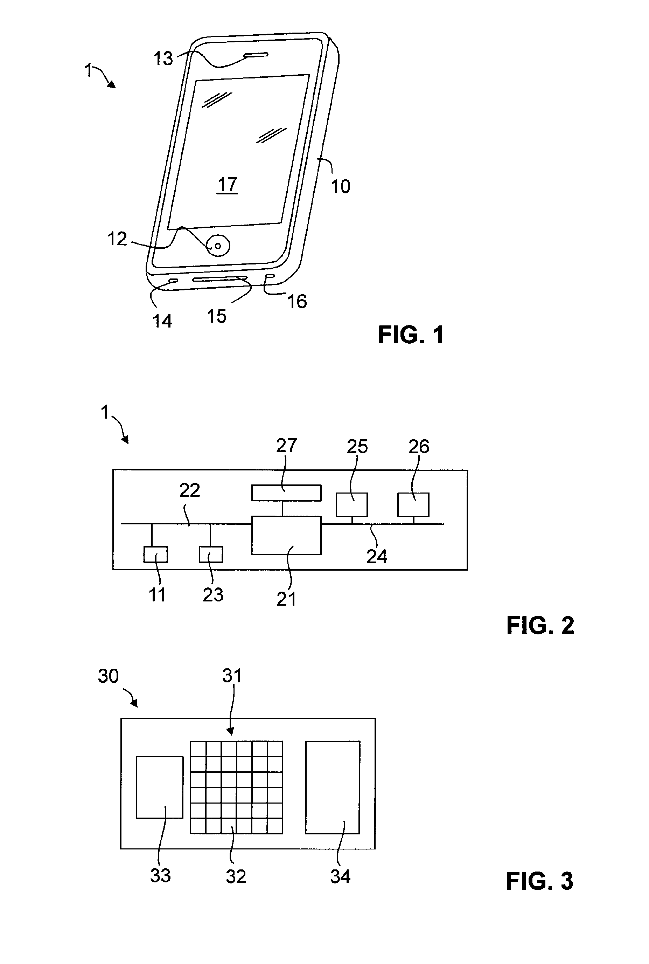

Figure 1 illustrates a portable electronic device in the form of a mobile phone 1. The mobile phone has a housing 10, an input/output device in the form of a touchscreen display 17 and a further input device in the form of a pushbutton 12. Below a first opening 13 in the front of the housing 10, an output device in the form of a loudspeaker is arranged. In a lower sidewall region of the housing 10, further openings 14, 15 and 16 are provided. Behind these openings, components such as a microphone, further loudspeakers and connectors are disposed. In addition, behind any of these openings sensors such as a humidity sensor, a temperature sensor and a sensor for detecting at least one chemical analyte (i.e., one or more chemical sensors) may be arranged. The chemical sensor may comprise one or more sensor cells, each sensor cell exhibiting a different sensitivity to selected analytes. The mobile phone runs an application program (app) or operating system service for operating the chemical sensor.

Figure 2 shows a schematic hardware-oriented block diagram of the mobile phone 1. A microprocessor 21 is connected via leads 22 to a chemical sensor 11 and at least one further sensor 23 (e.g., a humidity sensor, a temperature sensor, an inertial sensor etc.). The chemical sensor 11 contains signal processing capability in order to output a raw or preprocessed measured variable. A routine for analyzing the measured variable supplied by the chemical sensor 11 and outputting a result of the measurement may be executed by an evaluation unit. A hardware of the evaluation unit may be represented by the microprocessor 21, and a software of the evaluation unit may be represented by a program element stored in a memory 25 connected to the microprocessor 21 via a bus system 24. A short-range communication module 26, e.g. a Bluetooth module, and another wireless interface 27, e.g. a UMTS module or a WLAN module, may be connected to the microprocessor 21. Input/output devices as previously mentioned may further be connected to the microprocessor 21.

Hence, the present invention employs one or more chemical sensors that are sensitive to at least one chemical analyte. Each of these sensors may comprise one or more semiconductor sensor elements. These semiconductor sensor elements may comprise at least one sensitive layer, for which at least one electrical property (in particular, conductivity) changes in the presence of at least one chemical analyte due to adsorption and/or chemical reactions on the surface of the sensitive layer (including catalytic reactions in which the sensitive layer acts as a catalyst). The sensor may include at least one heat source integrated within the sensor to heat the sensitive layer to an operating temperature thereof. In particular, the sensitive layer may be a metal oxide (MOX) layer. Sensors having at least one MOX layer as a sensitive layer will in the following be called MOX sensors. The metal oxide may be, e.g., tin oxide, tungsten oxide, gallium oxide, indium oxide, or zinc oxide.

Each sensor may comprise two or more sensor elements ("cells") that have different sensitivities to selected analytes. The sensor cells may be arranged in a one- or two-dimensional array. Each sensor cell may provide a sensitive layer of a material exhibiting different sensitivity to some or all of the analytes that the sensor is sensitive to. For instance, each cell of the sensor array may specifically be mainly sensitive to a different analyte and as such may enable the portable electronic device to detect the presence or absence or concentration of such analyte. "Mainly" in this context shall mean that a sensor cell is more sensitive to the subject analyte than to other analytes. However, a sensor cell of such sensor array may exhibit not only sensitivity to its main analyte, but also to analytes other than the main analyte since such sensor cell may exhibit a cross-sensitivity to one or more analytes possibly representing main analytes for other cells. In this case, it is preferred that different sensor cells have different sensitivity profiles for the various analytes that the sensor is sensitive to. For instance, to discuss a particularly simple example, if one cell is sensitive to ethanol with a certain sensitivity and to acetone with a certain other sensitivity, it is preferred that another sensor cell is sensitive with a different ratio of sensitivities to ethanol and acetone, such that by comparing the signals of the two cells, the analytes ethanol and acetone can be separated.

The sensor cells may have different sensitivities to multiple different analytes at different operating conditions. For example, the sensor cell may mainly be receptive to a first analyte x when being heated to a first temperature Tx, and may mainly be receptive to a second analyte y when being heated to a second temperature Ty which is different from the first temperature Tx. To take advantage of this property, each of the sensor cells or specific groups of sensor cells may be provided with an individual heater. In other embodiments, all cells may be heated by the same heater. In some embodiments, the first and/or second sensor may comprise only a single sensor cell that has different sensitivities to multiple different analytes at different operating conditions.

In case the chemical sensor comprises more than one sensor element or sensor cell, the individual sensor cells may be embodied as discrete sensor cells. The sensor cells are preferably mounted on a common conductor board of the portable electronic device. The sensor cells may take the form of multiple chips. Each individual chip may be packaged, i.e. encapsulated, separately. In an alternative arrangement, multiple or all chips may be packaged in a common package, such that these chips are encapsulated by a common encapsulation. In a further embodiment, multiple or all sensor cells are monolithically integrated in a common sensor chip with a common substrate for multiple or all sensor cells. Such a monolithic multiple sensor chip may still be encapsulated and be arranged on and electrically connected to a conductor board of the portable electronic device.

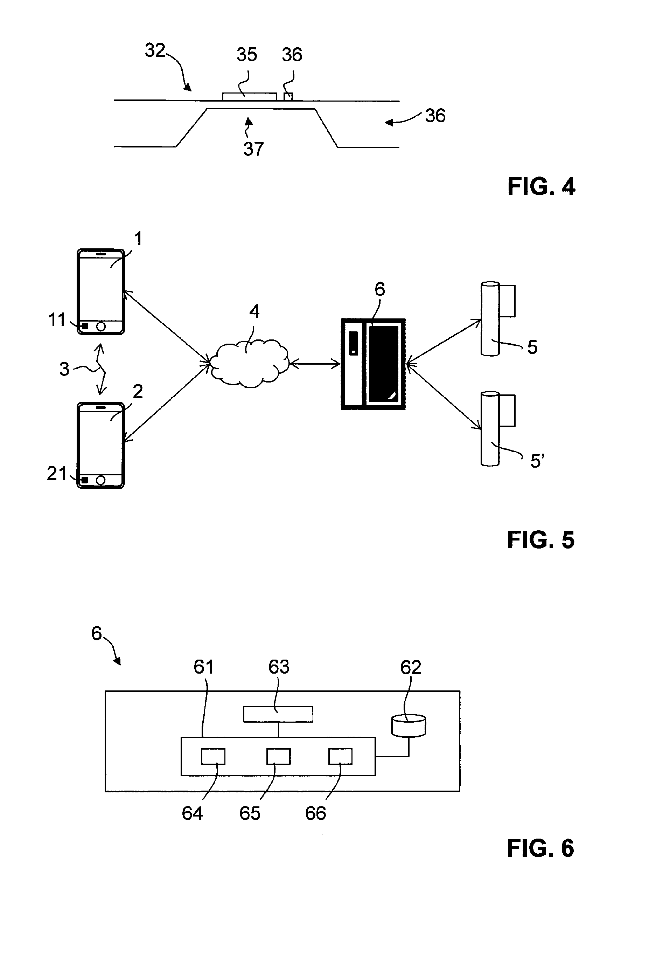

Figures 3 and 4 illustrate, in a highly schematic manner, an example of the sensor chip 30 implementing a chemical sensor as discussed above. The chip 30 comprises a chemical sensor structure 31 which takes the form of a sensor array comprising multiple sensor cells 32, in the present example, six times six sensor cells 32. In addition a humidity sensitive structure 33 is arranged next to the chemical sensor structure 32, and electronic circuitry 34 is integrated into the chemical sensor chip 30, which electronic circuitry 34 is responsible for linearizing and A/D converting the sensor signal and for outputting a measured variable. Figure 4 illustrates a cut through a schematic individual sensor cell 32. A recess is manufactured into a substrate 38 of the sensor chip to obtain a thin membrane 37. A sensitive layer 35 is arranged on top of the thin membrane, and a resistive heater 36 is arranged in or on top of the membrane. The membrane may be denoted as a micro-hotplate. The sensitive layer 35 is made of a metal oxide material. It is heated by the heater 36 prior to and during taking a sensor reading, so as to ensure that the temperature of the sensitive layer 35 is sufficient for having a catalytic reaction between the analyte/s and the sensitive layer 35 take place at a sufficient rate. As a result, an electrical conductivity of the sensitive layer 35 is modified. The operating temperature may vary subject to the material used from about 100 °C to about 450 °C.

However, the invention is not limited to MOX sensors. For instance, a sensor may be used that functions on an optical principle, i.e., an optical property of a sensor material may be modified such as its transmission rate, and this optical property is determined. Another possible measurement principle is a chemomechanical principle, in which a mass change upon absorption is transformed into a surface acoustic wave or into a cantilever resonance, for example.

Applications may include the detection of toxic gases, the detection of ethanol in a user's breath, the detection and/or identification of odors, and many more. Hence, the mobile phone equipped with the chemical sensor may in addition to its original function provide chemical information as to its environment. The user may learn about chemical substances and compositions present in the device's surroundings, and may use, transmit or else further analyze such information. Such information may be transmitted elsewhere and be used elsewhere, or the user himself/herself may benefit from the information provided by the chemical sensor. The electronic device may be primarily designed for computing and/or telecommunication and/or other tasks in the IT arena, but may be enhanced by the function of providing chemical information as to its environment.

For determining a corrective to an undesired drift of the transfer function of the sensor, calibration values may be provided. For determining such calibration values, reference readings may be taken by the chemical sensor from time to time, preferably in the absence of one or more analytes to which the chemical sensor is sensitive. In the alternative or in addition to this, calibration values may be determined based on operational data of the electronic device, e.g., heating data in case the chemical sensor includes a heater, and specifically the cumulative time the heater was activated or deactivated in a given period in time. For example, the less the heater was activated over a certain period in time, the more likely a drift has occurred such that the compensation value preferably is dimensioned to compensate for a larger drift. Instead or in addition to these criteria, calibration values may also be determined based on sensor readings from a sensor of the electronic device either different from the chemical sensor, or from a different sensor cell of the same chemical sensor comprising multiple sensor cells. Such sensor may be, e.g., a humidity sensor or a temperature sensor. For instance, it is known that certain poisonous gases negatively affect the drift behavior of a MOX sensor. The calibration value may then depend on the previous and/or present sensor measurements by a suitable function. For example, exposure to poisonous gases or infrequent sensor use might lead to sensor drift or loss of accuracy, which in turn requires a compensation value dimensioned to compensate for such influences on sensor performance.

A prediction model may be provided for predicting calibration values on the basis of past calibration values and possibly taking into account the sensor history. For instance, linear regression may be applied to past offset compensation values to extrapolate future offset compensation values.

However, this kind of procedure for determining and predicting calibration values may still lead to wrong results. In particular, the prediction model may fail if the sensor has not been operated for an extended period of time, or if the sensor has been exposed to a detrimental chemical environment, for instance to poisonous gases, for some time. Furthermore, while the above-described procedure may be well adapted to compensating offset drifts, sensitivity drifts cannot readily be detected and compensated by this procedure. Accordingly, it may be required to recalibrate the sensor from time to time by different methods. Recalibration may also be desired after the sensor has been reconditioned, e.g. by extended heating of the sensitive layer in case of a semiconductor sensor.

The present invention provides methods for recalibration. Exemplary embodiments of such methods and a corresponding system are illustrated in Figures 5 to 7.

Figure 5 illustrates a first mobile phone 1 equipped with a first chemical sensor 11 (shown only very schematically) and a second mobile phone 2 equipped with a second chemical sensor 21. Both mobile phones are equipped with a short-range communication module such as a Bluetooth module and with a network communication module such as a LAN module, as discussed in conjunction with Figure 2. The first mobile phone 1 executes an application program (app) or operating system service (henceforth called service) for operating its chemical sensor 11. The app or service monitors whether a second electronic device, e.g. the second mobile phone 2, is within the reach of the short-range communication module of the first mobile phone 1. When the app or service determines in this manner that a second electronic device is nearby, it requests context information from the second electronic device, for instance humidity data, temperature data, pressure data; acceleration data, magnetometer data, brightness data, image data, acoustic data and GPS data from the second electronic device. These data may be transmitted through the short-range communication interface 3 established by the short-range communication modules of the involved electronic devices, or the data may be transmitted through a network 4 to which both the first mobile phone 1 and the second electronic device are communicably attached, employing the network communication modules of the electronic devices. In other words, it is conceivable that the short-range communication interface 3 is only used for detecting proximity of the second electronic device, whereas the subsequent data communication is carried out through a network, which usually will have a higher bandwidth. The app or service then compares the context information received from the second electronic device with corresponding information derived from its own sensors. It is also conceivable that this comparison is not carried out by the app or service, but by a remote server 6 communicably connected to the network 4. If this comparison indicates that both the first mobile phone 1 and the second electronic device are likely to have the same chemical environment, the app or service starts a recalibration procedure as detailed below.

In parallel, the app or service periodically sends position information, network information and further context information such as accelerometer data, humidity data and temperature data via the network 4 to the remote server 6. The remote server 6 receives similar data from a plurality of further electronic devices. It determines the geolocation of each electronic device from the data and compares the geolocations of the electronic devices to determine whether any two or more electronic devices are near one another. In addition, the remote server 6 is in communication with a plurality of reference stations 5, 5' equipped with reference sensors. The geolocations of these reference stations are known to the server 6. The server 6 compares the geolocations of the electronic devices to the known geolocations of the reference stations to determine whether any electronic device is near one of the reference stations. If, for any specific electronic device, the server has determined that another electronic device or a reference station is nearby, it sends a corresponding message to the corresponding electronic device so as to start a recalibration procedure.

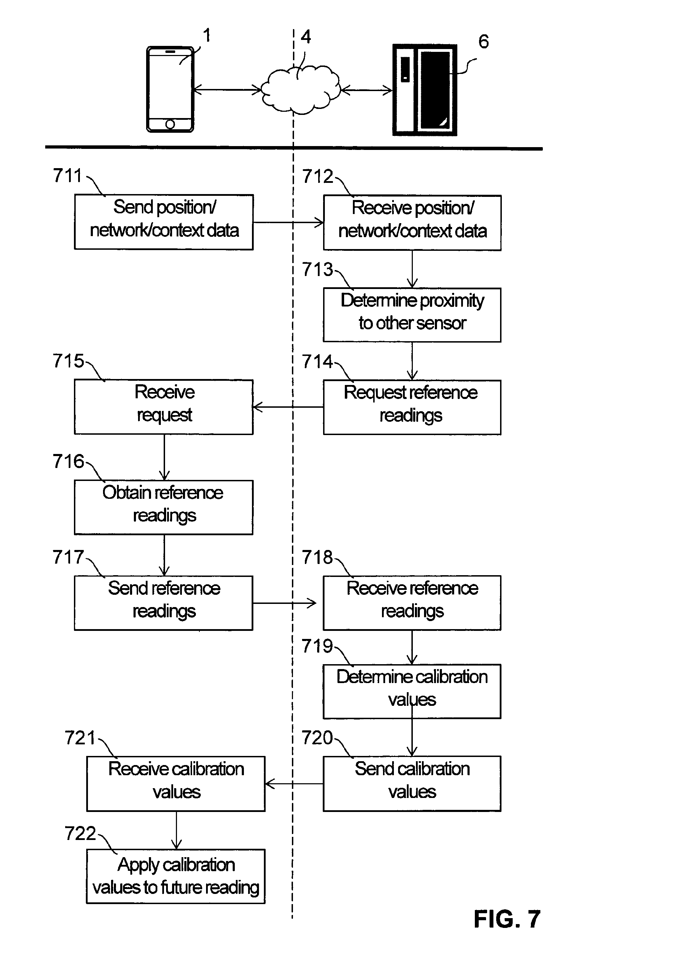

These steps and a subsequent recalibration procedure are illustrated in Figure 7. In step 711, the mobile phone 1 sends position, network and context data to the remote server 3 via the network 4. In step 712, the remote server 3 receives the position, network and context data. In step 713, the server 3 determines whether, for any given portable electronic device, another electronic device with another chemical sensor is nearby and is likely to have the same chemical environment. If this is the case, it starts the recalibration procedure.

A possible embodiment of a recalibration procedure comprises the subsequent steps 714 to 721. In step 714, the server requests readings from those electronic devices that have been determined to have the same chemical environment. Each electronic device receives the request in step 715 and obtains at least one reading in step 716. The reading may include the result of a recent measurement, and/or a new measurement may be triggered by the request. The reading may include results from one or more different cells of the chemical sensor. The reading is transmitted to the server in step 717 and is received by the server in step 718. Each reading may include an accuracy indicator, which may include parameters relating to the sensor identity and history.

In step 719, calibration values are derived from the reference readings. To this end, the accuracy indicators are compared. If this comparison shows that the expected accuracy of one of the sensors is higher than of the other sensors, the reading of that sensor may be assumed to be correct, and the calibration values of the other sensors may be set to such values that the readings of the other sensors correspond to the reading of the sensor having the highest expected accuracy. Of course, many other procedures for deriving calibration values are conceivable. A further plausibility check may be carried out at this stage by comparing the readings from sensor cells that are not subject to recalibration. If one or more of these readings indicate that the sensors have significantly different chemical environments, the procedure may still be stopped.

In step 720, the new calibration values are sent back to at least one of the electronic devices. They are received by electronic device in step 721 and are subsequently applied to future measurements in step 722. Instead, it is also possible to store the new calibration values in a remote database. In this case, whenever a measurement with a chemical sensor is carried out, the corresponding electronic devices would send their uncalibrated sensor readings to a remote determination unit, and the remote determination unit would apply the calibration values from the database to derive calibrated readings.

User intervention may be foreseen in various stages of the procedure. For instance, if it has been determined that the two electronic devices are near each other, the user may be requested to confirm that the electronic devices have the same chemical environment and are not exposed to any stimuli that might interfere with the calibration procedure. It is also conceivable that the user is requested to manually trigger a measurement by the chemical sensor of one or both of the involved electronic devices.

A highly schematic block diagram of a possible embodiment of a server is illustrated in Figure 6. The server has a processor 61, a memory 62 and a network communication module 63. The processor 61 executes a server program that has several software modules, including the following: a locating module 64 configured to receive data through the network communication module 63, to extract position, network and context data relating to at least one electronic device from the received data, and to determine a location of each device for which data is received, based on the received data; a matching module 65 configured to determine whether any two or more electronic devices are near one another; and a calibration module 66 configured to receive reference readings of the chemical sensors of the electronic devices that are near each other, and, subject to the reference readings, deriving calibration data for at least one of the electronic devices that are near each other.

## Claims

1. A method for calibrating a portable first electronic device (1) comprising a first chemical sensor (11), the method comprising:
determining whether the first electronic device (1) is located near a second electronic device (2) comprising a second chemical sensor (21);
comparing at least one reading of the first chemical sensor (11) and at least one reading of the second chemical sensor (21) obtained while the first electronic device (1) is near the second electronic device (2); and
subject to the comparison, deriving at least one calibration value for the first chemical sensor (11).

2. The method of claim 1, comprising:
triggering at least one of the first and second electronic devices (1, 2) to carry out a measurement with their chemical sensors (11, 21) when it is determined that the first electronic device (1) is located near the second electronic device (2).

3. The method of claim 1 or 2, wherein the determination whether the first electronic device (1) is located near the second electronic device (2) comprises:
receiving user input to at least one of the first electronic device (1) and the second electronic device (2), the user input indicating that the first electronic device (1) is located near the second electronic device (2).

4. The method of any one of the preceding claims, wherein each of the first electronic device (1) and the second electronic device (2) comprises a short-range communication module (26), and wherein the determination whether the first electronic device (1) is located near the second electronic device (2) comprises:
detecting whether the short-range communication module (26) of the first electronic device (1) and the short-range communication module of the second electronic device (2) are within an operating range of each other.

5. The method of any one of the preceding claims, wherein the first electronic device (1) comprises a network communication module (27) for communication with a network, and wherein determination whether the first electronic device (1) is located near the second electronic device (2) comprises:
transmitting data from the first electronic device (1) to a remote server (6), the data containing network and/or position information relating to the first electronic device (1);
on the remote server (6), determining a position of the first electronic device (1) based on the transmitted data.

6. The method of any one of the preceding claims, comprising:
obtaining context information about the first electronic device (1) and/or the second electronic device (2);
from the context information, determining whether the first and the second sensor (11, 21) are in substantially the same chemical environment; and
comparing at least one reading of the first chemical sensor (11) and at least one reading of the second chemical sensor (21) obtained while the first electronic device is near the second electronic device and while the first sensor (11) is substantially in the same chemical environment as the second sensor (21).

7. The method of claim 6, wherein the context information includes at least one of the following:
• humidity data;
• temperature data;
• pressure data;
• linear and/or rotational acceleration data;
• magnetometer data;
• brightness data;
• image data;
• acoustic data;
• data from at least one further chemical sensor of the first electronic device;
• position information; and
• network information about a network to which the first and/or electronic device is communicably attached.

8. The method of any one of the preceding claims, wherein each of the first chemical sensor (11) and the second chemical sensor (21) is assigned an accuracy indicator, and wherein the at least one calibration value for the first chemical sensor (11) is determined subject to the accuracy indicators of the first and second electronic sensors (11, 21).

9. The method of any one of the preceding claims, wherein the calibration value for the first chemical sensor (11) includes at least one of the following:
an offset parameter related to an offset reading in the absence of an analyte to which the first chemical sensor is sensitive; and
a sensitivity parameter related to a sensitivity of the first sensor to a concentration of at least one analyte to which the first chemical sensor is sensitive.

10. A method of determining calibration data for electronic devices (1; 2), each electronic device being equipped with at least one chemical sensor (11; 21), the method comprising:
receiving data through a network (4), the data containing network and/or position information relating to at least one electronic device (1; 2);
based on the received data, determining a location of each electronic device (1; 2) for which data is received;
determining whether any two or more electronic devices are in substantially the same chemical environment, in particular, located near one another;
receiving, through the network, readings of the chemical sensors of the electronic devices which are in substantially the same chemical environment;
subject to the readings, deriving calibration data for at least one of the electronic devices.

11. The method of claim 9, further comprising:
sending the derived calibration data to at least one of the electronic devices (1; 2) or to a remote determination unit communicably connected to at least one of the electronic devices via a network.

12. The method of any one of claims 9 to 11, wherein at least one of the electronic devices is a reference station equipped with a reference sensor.

13. A system for determining calibration data for electronic devices (1; 2) equipped with at least one chemical sensor (11; 21), the system comprising:
a network communication module (63) for communicably attaching the system to a network;
a locating module (64) configured to receive data through the network, the data containing network and/or position information relating to at least one electronic device (1; 2), and to determine a location of each electronic device (1; 2) for which data is received;
a matching module (65) configured to determine whether any two or more electronic devices (1; 2) are in substantially the same chemical environment, in particular, located near one another; and
a calibration module (66) configured to receive readings of the chemical sensors (11; 21) of the electronic devices (1; 2) that are in substantially the same chemical environment and, subject to the readings, to derive calibration data for at least one of the electronic devices (1; 2).

14. The system of claim 13, comprising a database (62) configured to store locations of the electronic devices.

15. A program element comprising computer code that, when executed in a processor, carries out the following method:
receiving data through a network (4), the data containing network and/or position information relating to at least one electronic device (1; 2);
based on the received data, determining a location of each electronic device (1; 2) for which data is received;
determining whether any two or more electronic devices (1; 2) are in the same chemical environment, in particular, located near one another;
receiving, through the network (4), readings of the chemical sensors (11; 21) of the electronic devices (1; 2) which are in the same chemical environment;
subject to the readings, deriving calibration data for at least one of the electronic devices (1; 2).
